(19) Europäisches Patentamt
European Patent Office
Office européen des brevets

(11) **EP 2 349 093 B1**

(12) **EUROPEAN PATENT SPECIFICATION**

(45) Date of publication and mention
of the grant of the patent:
**21.10.2015 Bulletin 2015/43**

(21) Application number: **09828192.6**

(22) Date of filing: **19.11.2009**

(51) Int Cl.:
**A61F 2/16** *(2006.01)*

(86) International application number:
**PCT/US2009/065049**

(87) International publication number:
**WO 2010/059764 (27.05.2010 Gazette 2010/21)**

(54) **DIFFRACTIVE MULTIFOCAL INTRAOCULAR LENS WITH MODIFIED CENTRAL DISTANCE ZONE**

DIFFRAKTIVE MULTIFOKALE INTRAOKULARLINSE MIT MODIFIZIERTER ZENTRALER FERNZONE

LENTILLE INTRAOCULAIRE MULTIFOCALE DIFFRACTIVE AVEC ZONE DE DISTANCE CENTRALE MODIFIÉE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **20.11.2008 US 116458 P**

(43) Date of publication of application:
**03.08.2011 Bulletin 2011/31**

(73) Proprietor: **Novartis AG**
**4056 Basel (CH)**

(72) Inventors:
• **SIMPSON, Michael**
**Arlington**
**Texas 76012 (US)**

• **VENKATESWARAN, Krishnakumar**
**Burleson**
**Texas 76028 (US)**

(74) Representative: **Hanna, Peter William Derek et al**
**Hanna Moore + Curley**
**13 Lower Lad Lane**
**Dublin 2, D02 T668 (IE)**

(56) References cited:
**EP-A1- 1 891 912        EP-A1- 2 045 648**
**WO-A1-2006/124198    US-A- 5 800 532**
**US-A1- 2006 116 764    US-A1- 2008 030 677**
**US-B2- 7 322 695**

**Description**

**BACKGROUND**

The present invention relates generally to multifocal ophthalmic lenses, and, more particularly, to multifocal intraocular lenses that can provide refractive and diffractive optical focusing powers.

Intraocular lenses (IOLs) are routinely implanted in patients' eyes during cataract surgery to replace a natural crystalline lens. Some IOLs employ diffractive structures to provide a patient with not only a far-focus power but also a near-focus power. In other words, such IOLs provide the patient with a degree of accommodation (sometimes referred to as "pseudoaccommodation"). The division of energy between the far-focus and the near-focus lens powers can be adjusted by modifying the "step heights" of the diffractive structure, and by the use of a central "refractive" zone that directs light solely to a single focus. An increase of energy to one focus generally causes a reduction of energy to the other focus, which reduces image contrast for that focus. However, image contrast is also affected by other factors, such as imaging aberrations, and the characteristics of the diffractive structure.

The publication WO2006124198 discloses a multifocal ophthalmic lens comprising a lens element having a central aspherical refractive zone for far focus and a diffractive bifocal zone disposed outside of the aspherical refractive zone, wherein said diffractive bifocal zone comprises a plurality of grooves, the grooves being apodized from a height directing light along a diffractive order associated with near focus to a height directing light along a diffractive order associated with far focus.

Accordingly, there is a need for diffractive multifocal lens designs that will enhance image contrast for both the far-focus and the near-focus.

**SUMMARY**

In one aspect, the present invention provides an intraocular lens (IOL), in accordance with claims which follow, which comprises an optic having an anterior surface and a posterior surface, where the optic includes a central refractive region for providing a refractive focusing power. A diffractive region is disposed on at least one of the lens surfaces for providing a near and far diffractive focusing power. In some cases, the refractive and diffractive far focusing powers are substantially equal. The optical properties of light passing through the central zone can be adjusted to optimize overall image contrast for both powers.

In a related aspect, in the above IOL, one of the surfaces (e.g., the anterior surface) includes a central refractive region that is surrounded by a diffractive region, which is in turn surrounded by an outer refractive region. In some cases, the central refractive region can have a diameter in a range of about 0.5 mm to about 2 mm.

In another aspect, the diffractive region includes a plurality of diffractive zones (e.g., 2 to 20 zones) that are separated from one another by a plurality of steps. The height of the central step, and/or the curvature of the central zone, are adjusted to optimize image contrast. The other steps exhibit substantially uniform heights.

In another aspect, a multifocal ophthalmic lens (e.g., an IOL) is disclosed, which includes an optic having an anterior surface and a posterior surface configured such that the optic includes a central refractive and an outer refractive region. In addition, a diffractive region is disposed on at least one of the surfaces to provide two diffractive focusing powers.

In some cases, in the above ophthalmic lens, the central and the outer refractive regions provide different refractive powers, e.g., the central region can provide a far-focusing power and the outer refractive region can provide a near-focusing power. The diffractive region can, in turn, provide diffractive near and far focusing powers corresponding to the refractive near and far focusing powers provided by the central and the outer regions.

In each of these aspects, the central refractive region of the embodiments of the ophthalmic lens of the present invention comprises a central distance zone, and the diffractive region can have reduced step heights, both cooperating to increase the amount of energy directed to the distance power of the IOL optic while maintaining an acceptable level of near-focusing power.

Further understanding of various aspects of the invention can be obtained by reference to the following detailed description in conjunction with the associated drawings, which are discussed briefly below.

**BRIEF DESCRIPTION OF THE DRAWINGS**

FIGURE 1 is a schematic side view of a prior art apodized diffractive multifocal IOL;

FIGURE 2A is a schematic top view of a multifocal IOL;

FIGURE 2B is a schematic side view of a the multifocal IOL shown in FIGURE 2A having a central distance zone with an adjusted central zone phase and approximately the same curvature as the base curve;

FIGURE 2C shows a radial profile of the anterior surface of the IOL shown in FIGUREs 2A and 2B from which the base profile of the anterior surface has been subtracted;

FIGURE 3 is a schematic side view of a multifocal IOL having a central distance zone with an adjusted central zone phase and central zone slope;

FIGURE 4 is a series of graphs illustrating the optical properties as a function of off-axis distance squared of embodiments of the present invention having different central zone phase and central zone curvature combinations;

FIGURE 5 is a series of graphs illustrating changes in the Modulation Transfer Function for embodiments of an IOL of the present invention having different central phase values;

FIGURE 6A is a schematic side view of a multifocal IOL having a reverse-apodized diffractive region;

FIGURE 6B is a radial profile of the anterior surface (minus the base profile of the surface) of the IOL shown in FIGURE 6A;

FIGURE 6C is a schematic side view of a multifocal IOL

FIGURE 6D is a radial profile of the anterior surface (minus the surface base profile) of the IOL of FIGURE 6C, indicating that the steps separating different diffractive zones of a diffractive region disposed on the surface exhibit an increase in heights followed by a decrease as a function of increasing radial distance from the lens center;

FIGURE 6E is a radial profile of a surface (minus the surface base profile) of an IOL which the steps separating different diffractive zones of a diffractive region disposed on the surface exhibit a decrease in heights followed by an increase as a function of increasing radial distance from the lens center;

FIGURE 7 is a radial profile of a surface (minus the surface base profile) of an IOL according to an embodiment of the invention in which the steps separating different diffractive zones of a diffractive region disposed on the surface exhibit substantially uniform heights;

FIGURE 8 is a schematic side view of an IOL according to an embodiment of the invention in which a diffractive region disposed on the lens's anterior surface extends to the periphery of the lens; and

FIGURE 9 is a schematic side view of an IOL according to an embodiment of the invention having a central refractive region and an outer refractive region, which provide different refractive focusing powers.

## DETAILED DESCRIPTION

The present invention generally provides multifocal ophthalmic lenses, e.g., multifocal intraocular lenses, that employ a refractive region for providing a refractive focusing power and a diffractive region for providing one or more diffractive focusing powers. In some cases the refractive focusing power provided by the lens corresponds to a far focus optical power that is substantially equal to one of the diffractive focusing powers while the other diffractive power corresponds to a near-focus optical power. As such, in some cases, the focusing properties of the lenses are dominated by their far-focus ability, especially for small pupil sizes. The salient features of various aspects of the invention are discussed in connection with intraocular lenses (IOLs). The teachings of the invention can also be applied to other ophthalmic lenses, such as contact lenses. The term "intraocular lens" and its abbreviation "IOL" are used herein interchangeably to describe lenses that are implanted into the interior of the eye to either replace the eye's natural lens or to otherwise augment vision regardless of whether or not the natural lens is removed. Intracorneal lenses and phakic intraocular lenses are examples of lenses that may be implanted into the eye without removal of the natural lens.

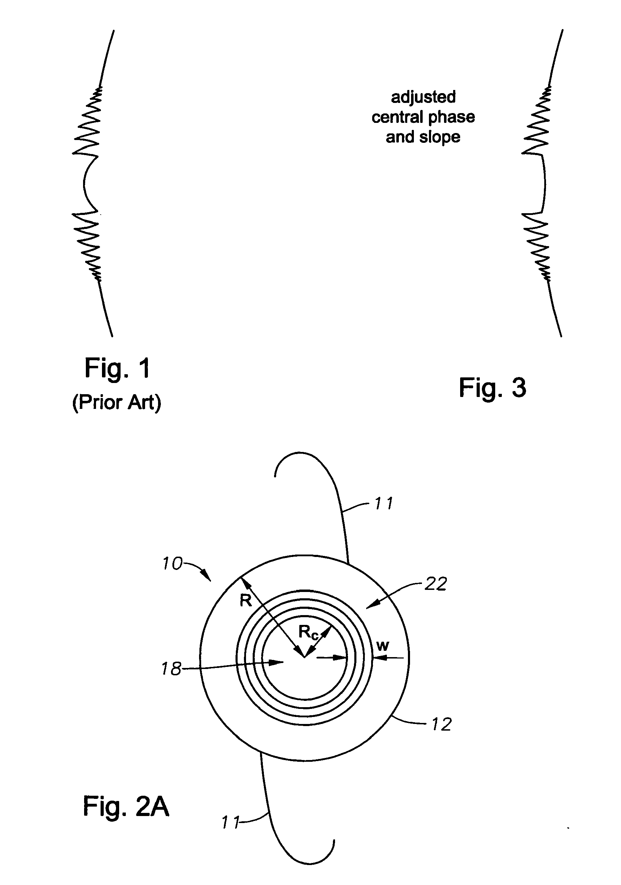

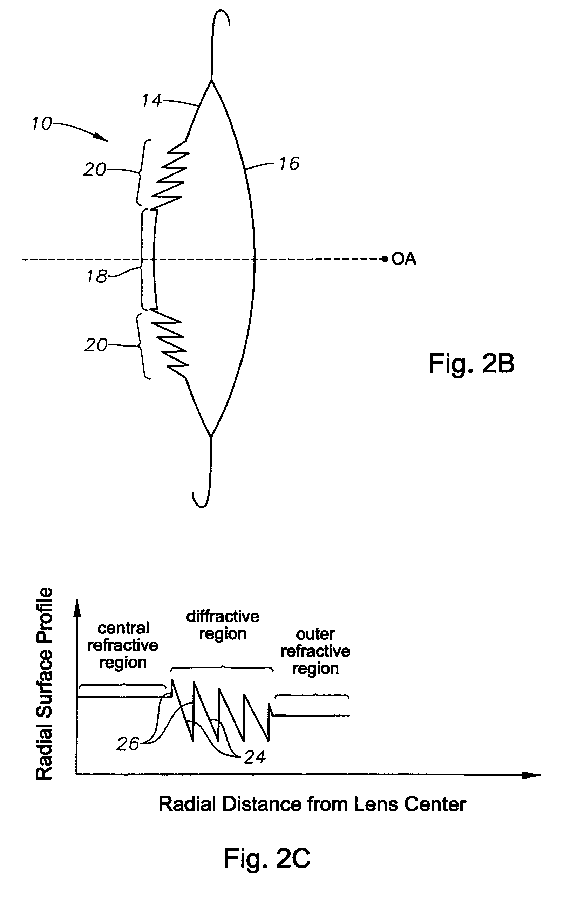

FIGURE 1 schematically depicts a prior art apodized diffractive multifocal IOL lens surface, where the curvature of the central zone is broadly similar to the curvature of the adjacent annular zone. FIGUREs 2A, 2B and 2C schematically depict a multifocal intraocular lens (IOL) 10 that includes an optic 12 having an anterior surface 14 and a posterior surface 16, which are disposed about an optical axis OA. As discussed in more detail below, the IOL 10 provides a far as well as a near focusing power. While the IOL has a biconvex profile (each of the anterior and posterior surfaces has a convex profile), the IOL can have any other suitable profile, e.g., convex-concave, plano-convex, etc. The optic 12 can have a maximum radius (R) from the optical axis OA in a range of about 2 mm to about 4 mm, while in other cases it can be larger. To direct more light to the distance focus, for example, all the step heights of the diffractive steps are reduced compared to the prior art example in FIGURE 1. This has the effect of directing more light to the distance focus and less light to the near focus.

In addition to changes in the diffractive step heights, the anterior surface 14 includes a central "refractive" region 18, which is surrounded by an annular diffractive region 20, and an outer refractive region 22. If the central region has a "refractive" focus that corresponds to the far power of the lens, then additional light is directed to that lens power. The central refractive region 18 can have a radius ($R_c$) relative to the optical axis OA in a range of about 0.25 mm to about 1 mm - though other radii can also be employed. The posterior surface 16 does not include any diffractive structures, though it can include such structures. As discussed further below, the central refractive region 18 of the anterior surface contributes to the focusing power of the optic, which corresponds in this embodiment to the IOL's far-focus optical power. By way of example, in some cases, the optic's distance power can be in a range of about -5 to about +55 Diopters and more typically in a range of about 6 to about 34 Diopters, or in a range of about 18 to about 26 Diopters.

In the example of FIGUREs 2A-2C, the base profiles of both the anterior surface 14 and the posterior surface 16 are substantially spherical with curvatures that are chosen, together with the index of refraction of the material forming the optic to provide a lens with the distance power only, in the absence of the diffractive structure. However, the axial location of the central zone region is adjusted so that it does not match the base curve. This is also indicated by the height separation between the central and outer regions in FIGURE 2C. This adjustment of the relative optical phase of the central zone, compared to the rest of the lens, can be used to adjust image contrast for both lens powers somewhat independently from the division of energy to the two foci. Similarly, the curvature of the surface of the central zone can also be adjusted, either alone or in conjunction with a phase delay at the diffractive step, in order to optimize the image contrast.

One or both lens surfaces can exhibit aspherical base profiles adapted to control aberrations and increase image contrast. By way of example, an IOL can comprise an optic having an anterior surface and a posterior surface. The anterior surface can include a refractive central region that generates, in cooperation with the posterior surface, a refractive optical power. Similar to the previous case, a diffractive region can surround the refractive central region. The diffractive region can, in turn, be surrounded by a refractive outer region. In such a case, the anterior surface has an aspheric base profile. In other words, the base profile of the anterior surface differs from a putative spherical profile. For example, the aspheric base profile of the anterior surface can be characterized by a negative conic constant, which can be selected based on the refractive power of the lens, that controls aberration effects. By way of example, the conic constant can be in a range of about -10 to about -1000 (e.g., -27). Though in this case, the base profile of the posterior surface is substantially spherical, in other cases, the base profile of the posterior surface can also exhibit a selected degree of asphericity such that the combined aspherical profiles of the two surfaces would facilitate the generation of a single refractive focus by the central portion of the lens. In other cases, the central refractive zone can have a spherical profile in order to facilitate the generation of a single refractive focus, even when the surface has an otherwise aspheric base profile.

Referring again to FIGUREs 2A, 2B and 2C, the optic 12 can be formed of any suitable biocompatible material. Some examples of such materials include, without limitation, soft acrylic, silicone, hydrogel or other biocompatible polymeric materials having a requisite index of refraction for a particular application of the lens. In many cases, the index of refraction of the material forming the optic can be in a range of about 1.4 to about 1.6 (e.g., the optic can be formed of a lens material commonly known as Acrysof® (a cross-linked copolymer of 2-phenylethyl acrylate and 2-phenylethyl methacrylate) having an index of refraction of 1.55)

The exemplary IOL 10 also includes a plurality of fixation members (e.g., haptics) 11 that facilitate placement of the IOL in a patient's eye. The fixation members 11 can also be formed of suitable polymeric materials, such as polymethylmethacrylate, polypropylene and the like.

As noted above, the optic 12 also includes a diffractive region 20, which is disposed on its anterior surface 14, though in other embodiments it can be disposed on the posterior surface or on both surfaces. The diffractive region 20 forms an annular region surrounding the central refractive region 18 of the optic's anterior surface. The diffractive region 20 provides a far-focus optical power as well as a near-focus power. In this example, the far-focus optical power provided by the diffractive structure is substantially similar to the refractive focusing power provided by the IOL's central refractive region. The near-focus optical power provided by the diffractive region can be, e.g., in a range of about 1 D to about 4 D, though other values can also be used. In some cases, the diffractive region 20 can have a width (w) in a range of about 0.5 mm to about 2 mm, though other values can also be employed. In other cases the diffractive region 20 can provide a far-focus optical power and not a near-focus power.

Although the diffractive region can extend to the outer boundary of the optic 12, in this embodiment, the diffractive region is truncated. More specifically, the diffractive region is disposed between the lens's central refractive region 18 and its outer refractive region 22. Similar to the refractive central region, the outer refractive region provides a single refractive focusing power, which in this case is substantially equal to the refractive power provided by the central region. In other words, the IOL's central and the outer refractive regions contribute only to the lens's far-focus power, while the diffractive region (herein also referred to as the zonal diffractive region) directs light energy incident thereon into both the far and near foci of the lens. As will be described herein, the energy directed to the far-focus power can be increased by reducing the diffractive region step heights and/or by adjusting the curvature of the central refractive distance zone.

As shown schematically in FIGURE 2C, which is a surface profile of the anterior surface without the base profile of the surface, the diffractive region 20 is formed of a plurality of diffractive zones 24 disposed on an underlying base curve of the anterior surface 14. The number of the diffractive zones can be in a range of about 2 to about 20, though other numbers can also be employed. The diffractive zones 24 are separated from one another by a plurality of steps 26. In this case, the heights of the steps 26 are non-uniform. More specifically, in this example, the step heights decrease as a function of increasing distance from a center of the anterior surface (the intersection of the optical axis OA with the anterior surface). In other words, the steps are apodized to exhibit decreasing heights as a function of increasing radial distance from the lens's optical axis. As discussed in more detail below, in other cases, the step heights can exhibit other types of non-uniformity, or alternatively, in embodiments of the invention, they can be uniform. The schematic radial profile depicted in FIGURE 2C also shows that the curvatures of the IOL's central and outer refractive regions correspond to the base curvature of the anterior surface (hence these regions are shown as flat in the figure), though a phase shift is given to the central zone. Other configurations, as described below, can also be used to divert more energy to the far-focus power of the embodiments of the present invention.

The steps are positioned at the radial boundaries of the diffractive zones. In this exemplary embodiment, the radial location of a zone boundary can be determined in accordance with the following relation:

$$r_i^2 = r_0^2 + 2i\lambda f \qquad \text{Equation (1)},$$

wherein

$i$ denotes the zone number
$r_0$ denotes the radius of the central refractive zone,
$\lambda$ denotes the design wavelength, and
$f$ denotes a focal length of the near focus.

In some embodiments, the design wavelength $\lambda$ is chosen to be 550 nm green light at the center of the visual response.

In some cases, the radius of the central zone ($r_0$) can be set to be $\sqrt{\lambda f}$.

With continued reference to FIGURE 2C, in some cases, the step height between adjacent zones, or the vertical height of each diffractive element at a zone boundary, can be defined according to the following relation:

$$\text{Step height} = \frac{\lambda}{2\,(n_2 - n_1)} f_{apodize} \qquad \text{Equation (2)},$$

wherein

$\lambda$ denotes the design wavelength (e.g., 550 nm),
$n_2$ denotes the refractive index of the material from which the lens is farmed,
$n_1$ denotes the refractive index of a medium in which the lens is placed,

and $f_{apodize}$ represents a scaling function whose value decreases as a function of increasing radial distance from the intersection of the optical axis with the anterior surface of the lens. For example, the scaling function can be defined by the following relation:

$$f_{apodize} = 1 - \left\{\frac{(r_i - r_{in})}{(r_{out} - r_{in})}\right\}^{exp}, r_{in} \le r_i \le r_{out} \qquad \text{Equation (3)},$$

wherein

$r_i$ denotes the radial distance of the $j^{th}$ zone,

$r_{in}$ denotes the inner boundary of the diffractive region as depicted schematically in FIGURE 2C,
$r_{out}$ denotes the outer boundary of the diffractive region as depicted schematically in FIGURE 2C, and
*exp* is a value chosen based on the relative location of the apodization zone and a desired reduction in diffractive element step height. The exponent *exp can* be selected based on a desired degree of change in diffraction efficiency across the lens surface. For example, exp can take values in a range of about 2 to about 6.

As another example, the scaling function can be defined by the following relation:

$$f_{apodize} = 1 - (\frac{r_i}{r_{out}})^3 \qquad \text{Equation (4),}$$

wherein

$r_i$ denotes the radial distance of the $i^{th}$ zone, and
$r_{out}$ denotes the radius of the apodization zone.

Referring again to FIGURE 2C, each step at a zone boundary is centered about the base profile with half of its height above the base profile and the other half below the profile, apart from the central step. Further details regarding selection of the step heights, other than the height of the central step, can be found in U.S. Patent No. 5,699,142.

In use, the central refractive region provides a single far focus refractive power such that the IOL 10 effectively functions as a monofocal refractive lens for small pupil sizes, that is the pupil sizes less than or equal to the radial size of the central refractive region. For larger pupil sizes, while the central region continues to provide a single far-focus optical power, the diffractive region begins to contribute to the IOL's focusing power by providing two diffractive focusing powers: one substantially equal to the refractive far-focus power of the central region and the other corresponding to a near-focus power. As the pupil size increases further, the outer refractive region 22 can also contribute - refractively - to the far-focus power of the lens. The fraction of the light energy distributed to the near focus relative to the far focus can be adjusted, e.g., via the sizes of the central and outer refractive regions as well as the parameters (e.g., step heights) associated with the diffractive region. Further, in cases in which the step heights are apodized, this fraction can change as a function of the pupil size. For example, the decrease in the step heights of the diffractive structure results in an increase in the fraction of the light energy transmitted to the far focus by the diffractive structure as the pupil size increases.

The energy directed to the far-focus power of the embodiments of the present invention can thus be increased by reducing the diffractive step heights in the diffractive region and/or by adjusting the central distance zone curvature. The central zone could have the same curvature as the base curvature of the IOL, resulting in a simple central distance zone, or could be enhanced for improved far-focus performance by having a different central zone curvature. For example, FIGURE 2B shows a schematic side view of the multifocal IOL of FIGURE 2A having a central distance zone with an adjusted central zone phase and approximately the same curvature as the base curve. In this embodiment, the central zone phase is adjusted by adjusting (reducing) the height of the first diffractive step (the step closest to the central zone) and thereby changing the phase delay of the central zone. In another embodiment, such as shown in FIGURE 3, a multifocal IOL in accordance with the present invention is depicted having a central distance zone with an adjusted central zone phase and a central zone curvature adjusted to differ from that of the IOL base curve to control the image quality for both lens powers.

As can be seen from FIGUREs 2B and 3, and other FIGUREs herein, different lens parameters can be used alone or in combination to increase the energy distributed to the far-focus power of the embodiments of the present invention, and to control the image contrast. A central diffractive step adjustment can thus be combined with a change in central zone curvature, for example. The shape of the central zone can also be adjusted, and can be spherical or aspheric and differ from that of the base curve. Further, the adjustments to the central zone phase and curvature described herein can likewise be used to increase the energy distributed to the near-focus power in some embodiments as opposed to the far-focus power. Thus, the embodiments of the present invention can quite effectively be used to direct more energy to a first (far-focus) lens power or to a second (near-focus) lens power, while controlling image quality.

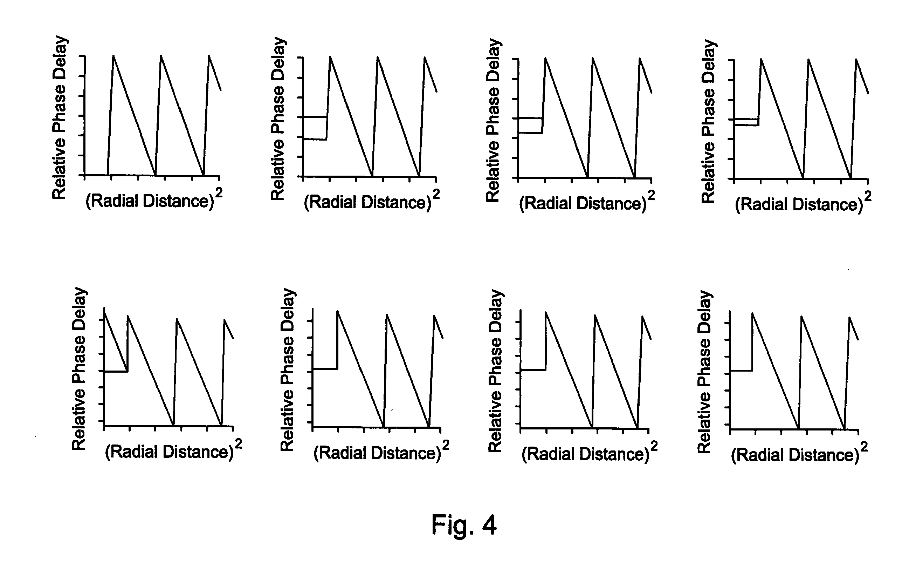

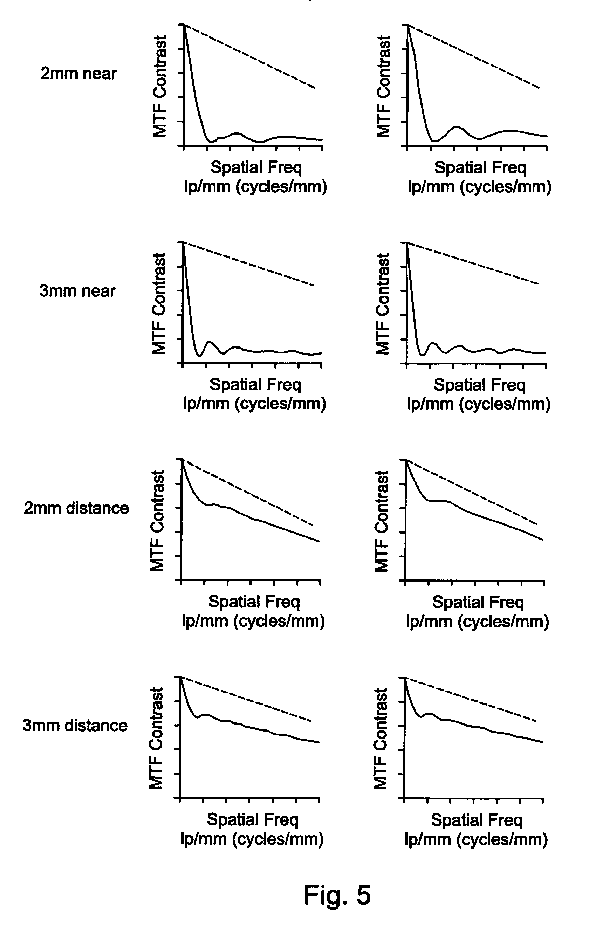

FIGURE 4 is a series of graphs illustrating the optical properties as a function of off-axis distance squared of embodiments of the present invention having different central zone phase and central zone curvature combinations. FIGURE 5 is a series of graphs illustrating changes in the Modulation Transfer Function (MTF) for embodiments of an IOL of the present invention having different central phase values. These graphs illustrate examples of adjustments to the central zone when plotted as a function of the square of the IOL radius. They represent the optical phase delay at the surface of the central zone and also the physical surface profile of the IOL optic. FIGURE 5 illustrates the improvements in far-focus power by a specific example of a prior art lens design having a simple central distance zone compared to an embodiment of an IOL of the present invention having a phase delay of 0.5 at the central diffractive step. In addition, this example shows increased energy to the far-focus power as compared to the prior art lens by reducing all of the diffractive region step heights. In this example, the MTF contrast increases for the near power with the introduction of the central zone phase delay, compared to a similar lens where there is no phase delay for the central zone.

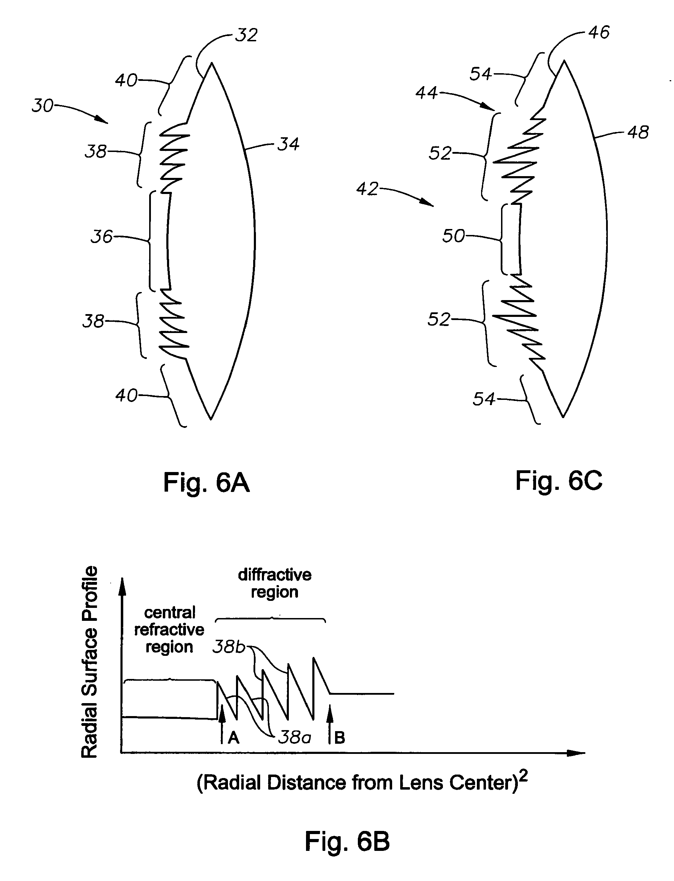

The apodization of the diffractive region is not limited to the one discussed above. In fact, a variety of types of apodization of the step heights can be employed. By way of example, with reference to FIGUREs 6A and 6B, in some embodiments, an IOL 30 can include an anterior surface 32 and a posterior surface 34, where the anterior surface is characterized by a central refractive region 36, an annular diffractive region 38 that surrounds the central refractive region 34, and an outer refractive region 40. The annular diffractive region is formed by a plurality of diffractive zones 38a that are separated from one another by a plurality of steps 38b, where the steps exhibit increasing heights from an inner boundary A of the diffractive region to an outer boundary B thereof.

Such an apodization of the step heights is herein referred to as "reverse apodization." Similar to the previous case, the diffractive region contributes not only to the IOL's far-focus optical power but also to its near-focus power, e.g., the near-focus power can be in a range of about 1 to about 4 D. However, unlike the previous case, the percentage of the incident light energy transmitted by the diffractive region to the far focus decreases as the pupil size increases (due to the increase in the step heights as a function of increasing radial distance from the optical axis).

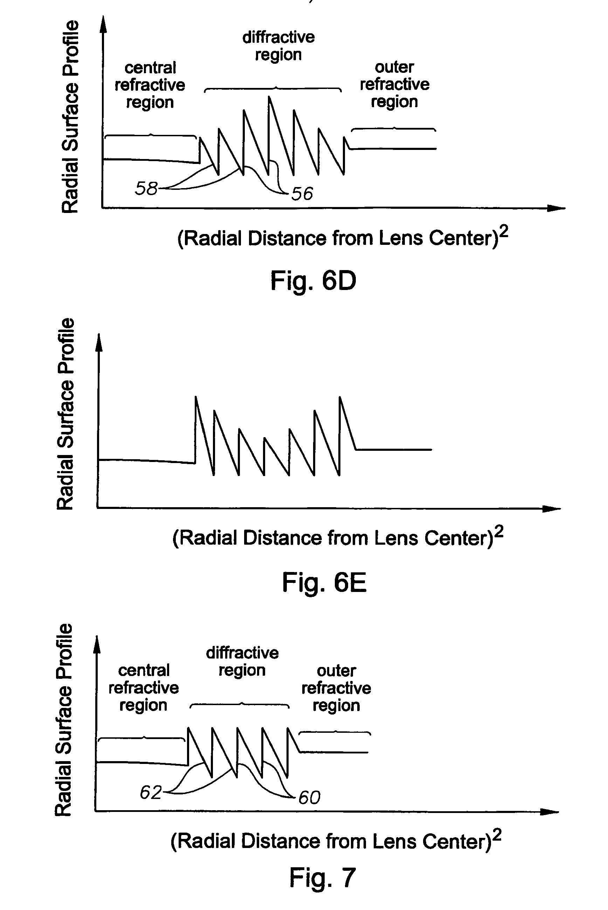

In other cases, the step heights in the diffractive region can increase from the region's inner boundary to reach a maximum value at an intermediate location within that region followed by a decrease to the region's outer boundary. By way of example, FIGURE 6C depicts such an IOL 42 having an optic 44 characterized by an anterior surface 46 and a posterior surface 48. Similar to the previous cases, the anterior surface 46 includes a central refractive region 50, an annular diffractive region 52 that surrounds the refractive region, and an outer refractive region 54 that in turn surrounds the diffractive region. With reference to the radial profile of the anterior surface presented in FIGURE 6D, the annular diffractive region includes a plurality of diffractive zones 56 separated from another by a plurality of steps 58, where the step heights exhibit an increase followed by a decrease as a function of increasing radial distance from the center of the lens. Alternatively, in another case shown schematically in FIGURE 6E, the step heights show a decrease followed by an increase as a function of increasing distance from the lens center.

In embodiments as claimed, the step heights separating different zones of the diffractive region can be substantially uniform (e.g., within manufacturing tolerances). By way of illustration, FIGURE 7 schematically depicts a radial profile of a surface of such a lens (e.g., the anterior surface of the lens) from which the underlying base profile has been subtracted. The radial surface profile indicates that the surface includes a central refractive region A (with a curvature that is substantially equal to the base curvature of the surface, but with an additional phase delay), a diffractive region B and an outer refractive region C. The diffractive region B is characterized by a plurality of diffractive zones 60 that are separated from one another by a plurality of steps 62. The heights of the steps 62 are substantially uniform.

By way of example, in some implementations of an IOL having a substantially uniform step height, which provides a selected phase shift at each zone boundary, the radial location of a zone boundary can be determined in accordance with equation 1. In some cases, the radius of the central zone ($r_0$) can be set to be $\sqrt{\lambda f}$. Further, the step height between adjacent zones can be defined in accordance with the following relation:

$$\text{Step height} = \frac{b\lambda}{(n_2 - n_1)} \qquad \text{Eq. (6)}$$

wherein

$\lambda$ denotes the design wavelength (e.g., 550 nm),
$n_2$ denotes the refractive index of the material from which the lens is formed,
$n_1$ denotes the refractive index of the medium in which the lens is placed, wand
$b$ is a fraction, e.g., 0.5 or 0.7.

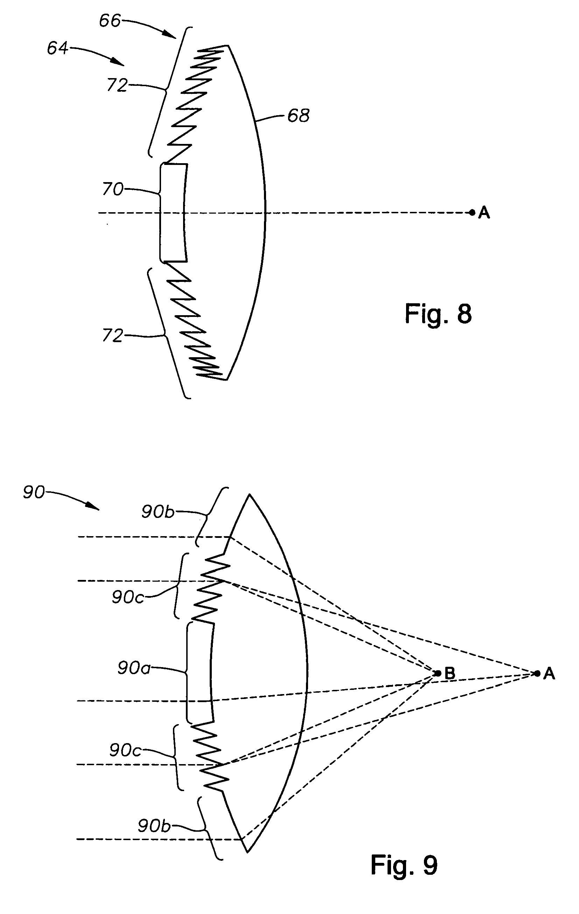

In some embodiments, the diffractive region can extend from the outer boundary of the central refractive region to the outer boundary of the optic. By way of example, FIGURE 8 schematically depicts such an IOL 64 that includes an anterior surface 66 and a posterior surface 68. The anterior surface includes a central refractive region 70 that, in cooperation with the refractive posterior surface, imparts to the optic a refractive far-focus power. The central zone has an adjustment in step height and/or curvature. A diffractive region 72 disposed on the anterior surface extends from the outer boundary of the central refractive region to the outer boundary of the optic, and provides a diffractive near-focus and a diffractive far-focus optical power. In this exemplary implementation, the diffractive far-focus power is substantially equal to the refractive far-focus power provided by the optic's refractive central region. In this example the diffractive region is formed by a plurality of diffractive zones separated by steps having substantially uniform heights.

In some other embodiments, an IOL can include a central refractive region, an annular diffractive region disposed on a surface thereof, and an outer refractive region, where the central and the outer refractive regions provide different refractive focusing powers. The central zone has an adjustment in step height and/or curvature. By way of example, as shown schematically in FIGURE 9, a central refractive region 90a of such an IOL 90 can contribute to the IOL's far-focus optical power (corresponding to far focus A) while an outer refractive region 90b of the IOL contributes - refractively - to the IOL's near-focus optical power (corresponding to near focus B). A diffractive region 90c, in turn, contributes -diffractively - to both the near and the far focusing powers of the IOL. Such a difference in the refractive focusing properties of the central and outer regions can be achieved, e.g., by configuring the outer region of one or both of the lens surfaces to have a different surface curvature (surface profile) than that of the respective central region.

In some cases, the base profile of at least one of the lens surfaces can exhibit a selected degree of asphericity to control aberrations, such as to control depth-of-focus. For example, the anterior surface on which a diffractive region is disposed can exhibit a spherical profile while the posterior surface exhibits a certain degree of asphericity. By way of example, further teachings regarding configuring one or more of the lens surfaces to have aspherical profiles can be found in pending U.S. patent application entitled "Intraocular Lens" having a serial number 11/397332, filed on April 4, 2006.

In other cases, at least one of the lens surfaces can have a toric base profile (a profile characterized by two different curvatures along two orthogonal directions of the surface) to help correct astigmatism.

In some embodiments, the biocompatible polymeric material of the optic can be impregnated one or more dyes such that the lens can provide some degree of filtering of blue light. Some examples of such dyes are provided in U.S. Patent Nos. 5,528,322 (entitled "Polymerizable Yellow Dyes And Their Use In Ophthalmic Lenses"), 5,470,932 (entitled "Polymerizable Yellow Dyes And Their Use In Ophthalmic Lenses"), 5,543,504 (entitled "Polymerizable Yellow Dyes And Their Use In Ophthalmic Lenses), and 5,662,707 (entitled "Polymerizable Yellow Dyes And Their Use In Ophthalmic Lenses).

A variety of known manufacturing techniques can be employed to form an ophthalmic lens (e.g., an IOL) in accordance with the teachings of the invention. For example, such techniques can be employed to initially form a refractive optic and subsequently generate an annular diffractive region on one of the surfaces of the optic such that the diffractive region would surround a central refractive region of the surface.

Those having ordinary skill in the art will appreciate the certain modifications can be made to the above embodiments without departing from the scope of the invention.

## Claims

1. An intraocular lens (IOL) (10,30,42,64), comprising
an optic (12) having an anterior surface (14) and a posterior surface (16), said optic having a central refractive region (18) for providing one refractive focusing power, and
a diffractive region (20) disposed on one of said surfaces so as to provide a near and a far diffractive focusing powers, wherein the diffractive region comprises a plurality of diffractive zones (24) separated from one another by a plurality of steps (26),
**characterized in that** the first innermost step differs in height from the second and subsequent step heights (60), which are of substantially uniform step height, so as to alter the phase of the central refractive region to provide increased light to the refractive focusing power.

2. The IOL of claim 1, wherein each of said anterior and posterior surfaces (14,16) includes a central refractive region (18).

3. The IOL of claim 2, wherein said central refractive region (18) of any of the anterior and the posterior surface has a diameter in a range of about 0.5 mm to about 2 mm.

4. The IOL of claim 2, wherein said central refractive region (18) of each of said anterior and the posterior surfaces (14,16) has a substantially spherical profile.

5. The IOL of claim 4, wherein said diffractive region (20) outside of said central refractive region (18) has a substantially aspheric base profile.

6. The IOL of claim 2, wherein the diffractive region (20) at least partially surrounds the central refractive region (18) of the surface on which it is disposed.

7. The IOL of claim 1, wherein said far focusing power of the diffractive region (20) corresponds substantially to said refractive focusing power provided by the optic's central refractive region (18).

8. The IOL of claim 1, wherein said optic (12) comprises an outer refractive region (22).

9. The IOL of claim 8, wherein said outer refractive region (22) provides a focusing power substantially equal to the refractive focusing power provided by the central refractive region (18).

10. The IOL of claim 1, wherein at least one of said surfaces (14,16) exhibits an aspheric base profile adapted to control aberrations of the lens.

11. The IOL of claim 1, wherein said central refractive region (18) has a substantially spherical profile.

12. The IOL of claim 11, wherein said diffractive region (20) outside of said central refractive region (18) has a substantially aspheric base profile.

13. A method of manufacturing an ophthalmic lens (10), comprising
forming an optic (12) having an anterior surface (14) and a posterior surface (16) having base profiles adapted for generating a far-focus; and
generating a diffractive structure (24,26) on at least one of said surfaces such that said surface comprises a central refractive region (18) and an outer refractive region (20), said diffractive structure contributing to said far-focus optical power while also providing a near-focus optical power, wherein the diffractive structure comprises a plurality of diffractive zones (24) separated from one another by a plurality of steps (26) and wherein the first innermost step differs in height from the second and subsequent step heights (60), which are of substantially uniform step height, so as to alter the phase of the central refractive region to provide increased light to the refractive focusing power.

**Patentansprüche**

1. Intraokularlinse (IOL) (10, 30, 42, 64), aufweisend
eine Optik (12) mit einer vorderen Fläche (14) und einer rückwärtigen Fläche (16), welche Optik einen zentralen refraktiven Bereich (18) zum Vorsehen einer refraktiven Fokussier-Stärke hat, sowie
einen diffraktiven Bereich (20), angeordnet an einer der beiden besagten Flächen, um so jeweils eine nah- und eine ferndiffraktive Fokussier-Stärke vorzusehen, wobei der diffraktive Bereich eine Mehrzahl an diffraktiven Zonen (24) aufweist, die voneinander durch eine Mehrzahl an Stufen (26) separiert sind,
**dadurch gekennzeichnet, dass** die erste innerste Stufe in ihrer Höhe von der zweiten und nachfolgenden Stufenhöhe(n) (60) differiert, die von im Wesentlichen gleicher Stufenhöhe sind, umso die Phase des zentralen refraktiven Bereiches zu modifizieren, um vermehrt Licht für die refraktive Fokussierleistung zuzuführen.

2. IOL nach Anspruch 1, wobei die jeweilige Fläche der vorderen und rückwärtigen Fläche (14, 16) einen zentralen refraktiven Bereich (18) aufweisen.

3. IOL nach Anspruch 2, wobei der zentrale refraktive Bereich (18) von einer der beiden vorderen oder rückwärtigen Fläche einen Durchmesser in einem Bereich von ca. 0,5 mm bis ca. 2 mm hat.

4. IOL nach Anspruch 2, wobei der zentrale refraktive Bereich (18) von jeder jeweiligen Fläche der vorderen und der rückwärtigen Fläche (14, 16) ein im Wesentlichen sphärisches Profil hat.

5. IOL nach Anspruch 4, wobei der diffraktive Bereich (20) außerhalb des besagten zentralen refraktiven Bereichs (18) ein im Wesentlichen asphärisches Basisprofil hat.

6. IOL nach Anspruch 2, wobei der diffraktive Bereich (20) zumindest teilweise den zentralen refraktiven Bereich (18) derjenigen Fläche umgibt, auf der er angeordnet ist.

7. IOL nach Anspruch 1, wobei die Fern-Fokussierleistung des diffraktiven Bereichs (20) im Wesentlichen der besagten refraktiven Fokussierleistung entspricht, die durch den zentralen refraktiven Bereich (18) der Optik vorgesehen ist.

8. IOL nach Anspruch 1, wobei die Optik (12) einen äußeren refraktiven Bereich (22) aufweist.

**9.** IOL nach Anspruch 8, wobei der besagte äußere refraktive Bereich (22) eine Fokussierleistung schafft, die im Wesentlichen der refraktiven Fokussierleistung entspricht, wie sie durch den zentralen refraktiven Bereich (18) vorgesehen ist.

**10.** IOL nach Anspruch 1, wobei mindestens eine Fläche der besagten Flächen (14, 16) ein asphärisches Basisprofil aufzeigt, dass dazu bestimmt ist, Aberrationen der Linse zu steuern.

**11.** IOL nach Anspruch 1, wobei der zentrale refraktive Bereich (18) ein im Wesentlichen sphärisches Profil hat.

**12.** IOL nach Anspruch 11, wobei der diffraktive Bereich (20) außerhalb des besagten zentralen refraktiven Bereichs (18) ein im Wesentlichen asphärisches Basisprofil hat.

**13.** Verfahren zur Herstellung einer ophthalmischem Linse (10), umfassend:

Bilden einer Optik (12) mit einer vorderen Fläche (14) und einer rückwärtigen Fläche (16) mit Basisprofilen, die zur Erzeugung eines Fern-Fokus bestimmt sind; und
Generieren einer diffraktiven Struktur (24, 26) auf zumindest einer der besagten Flächen, derart, dass die besagte Fläche einen zentralen refraktiven Bereich (18) und einen äußeren refraktiven Bereich (20) aufweist, wobei besagte diffraktive Struktur zur besagten optischen Fern-Fokusleistung beiträgt, während ebenso eine optische Nah-Fokusleistung vorgesehen ist, wobei die refraktive Struktur eine Mehrzahl an diffraktiven Zonen aufweist, die voneinander durch eine Mehrzahl an Stufen (26) separiert sind, und wobei die erste innerste Stufe in ihrer Höhe von der zweiten und nachfolgenden Stufenhöhe(n) (60) differiert, die von im Wesentlichen gleicher Stufenhöhe sind, umso die Phase des zentralen refraktiven Bereiches zu modifizieren, um vermehrt Licht für die refraktive Fokussierleistung zuzuführen.

## Revendications

**1.** Lentille intraoculaire (LIO) (10, 30, 42, 64), comprenant :

une optique (12) comportant une surface antérieure (14) et une surface postérieure (16), ladite optique comportant une région de réfraction centrale (18) pour fournir une puissance de focalisation de réfraction, et
une région de diffraction (20) disposée sur l'une desdites surfaces de manière à fournir des puissances de focalisation de diffraction de vision proche et de vision lointaine, dans laquelle la région de diffraction comprend une pluralité de zones de diffraction (24) séparées les unes des autres par une pluralité de gradins (26), **caractérisée en ce que** la hauteur du premier gradin le plus à l'intérieur diffère des hauteurs du deuxième gradin et des suivants (60), qui ont une hauteur de gradin sensiblement uniforme, de manière à modifier la phase de la région de réfraction centrale pour fournir davantage de lumière pour la puissance de focalisation de réfraction.

**2.** LIO selon la revendication 1, dans laquelle chacune desdites surfaces antérieure et postérieure (14, 16) comprend une région de réfraction centrale (18).

**3.** LIO selon la revendication 2, dans laquelle ladite région de réfraction centrale (18) de l'une quelconque des surfaces antérieure et postérieure a un diamètre dans une plage d'environ 0,5 mm à environ 2 mm.

**4.** LIO selon la revendication 2, dans laquelle ladite région de réfraction centrale (18) de chacune desdites surfaces antérieure et postérieure (14, 16) a un profil sensiblement sphérique.

**5.** LIO selon la revendication 4, dans laquelle ladite région de diffraction (20) à l'extérieur de ladite région de réfraction centrale (18) a un profil de base sensiblement asphérique.

**6.** LIO selon la revendication 2, dans laquelle la région de diffraction (20) entoure au moins partiellement la région de réfraction centrale (18) de la surface sur laquelle elle est disposée.

**7.** LIO selon la revendication 1, dans laquelle ladite puissance de focalisation de vision lointaine de la région de diffraction (20) correspond sensiblement à ladite puissance de focalisation de réfraction fournie par la région de réfraction centrale (18) de l'optique.

**8.** LIO selon la revendication 1, dans laquelle ladite optique (12) comprend une région de réfraction externe (22).

**9.** LIO selon la revendication 8, dans laquelle ladite région de réfraction externe (22) fournit une puissance de focalisation sensiblement égale à la puissance de focalisation de réfraction fournie par la région de réfraction centrale (18).

**10.** LIO selon la revendication 1, dans laquelle au moins l'une desdites surfaces (14, 16) présente un profil de base asphérique adapté pour contrôler les aberrations de la lentille.

**11.** LIO selon la revendication 1, dans laquelle ladite région de réfraction centrale (18) a un profil sensiblement sphérique.

**12.** LIO selon la revendication 11, dans laquelle ladite région de diffraction (20) à l'extérieur de ladite région de réfraction centrale (18) a un profil de base sensiblement asphérique.

**13.** Procédé de fabrication d'une lentille ophtalmique (10), comprenant :

la formation d'une optique (12) comportant une surface antérieure (14) et une surface postérieure (16) ayant des profils de base adaptés pour générer une focalisation de vision lointaine ; et
la génération d'une structure de diffraction (24, 26) sur au moins l'une desdites surfaces de sorte que ladite surface comprenne une région de réfraction centrale (18) et une région de réfraction externe (20), ladite structure de diffraction contribuant à ladite puissance optique de focalisation de vision lointaine tout en fournissant également une puissance optique de focalisation de vision proche, dans lequel la structure de diffraction comprend une pluralité de zones de diffraction (24) séparées les unes des autres par une pluralité de gradins (26), et dans lequel la hauteur du premier gradin le plus à l'intérieur diffère des hauteurs du deuxième gradin et des suivants (60), qui ont une hauteur de gradin sensiblement uniforme, de manière à modifier la phase de la région de réfraction centrale pour fournir davantage de lumière à la puissance de focalisation de réfraction.

**Fig. 1**
(Prior Art)

adjusted
central phase
and slope

**Fig. 3**

**Fig. 2A**

10

14

20

16

18

OA

20

## Fig. 2B

Radial Surface Profile

central
refractive
region

diffractive
region

outer
refractive
region

26

24

Radial Distance from Lens Center

## Fig. 2C

Fig. 4

**2mm near**

MTF Contrast

Spatial Freq
lp/mm (cycles/mm)

MTF Contrast

Spatial Freq
lp/mm (cycles/mm)

**3mm near**

MTF Contrast

Spatial Freq
lp/mm (cycles/mm)

MTF Contrast

Spatial Freq
lp/mm (cycles/mm)

**2mm distance**

MTF Contrast

Spatial Freq
lp/mm (cycles/mm)

MTF Contrast

Spatial Freq
lp/mm (cycles/mm)

**3mm distance**

MTF Contrast

Spatial Freq
lp/mm (cycles/mm)

MTF Contrast

Spatial Freq
lp/mm (cycles/mm)

## Fig. 5

**Fig. 6A**

**Fig. 6C**

**Fig. 6B**

Fig. 6D

Fig. 6E

Fig. 7

66

64

72

68

70

72

●A

**Fig. 8**

90

90b

90c

90a

90c

90b

●B

●A

**Fig. 9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006124198 A
- US 5699142 A
- US 11397332 B
- US 5528322 A
- US 5470932 A
- US 5543504 A
- US 5662707 A